# EUROPEAN PATENT SPECIFICATION

(11) **EP 4 175 557 B1**
(45) Date of publication and mention of the grant of the patent: **13.08.2025**
(21) Application number: 21746270.4
(22) Date of filing: 30.06.2021
(51) Int. Cl.: A61B 10/00

(54) **FLUID SAMPLE COLLECTION CONTAINER WITH CAP AND REMOVAL TOOL FOR FINGER GRIP LUER ADAPTER**
FLÜSSIGKEITSPROBEN-SAMMELBEHÄLTER MIT KAPPE UND ENTFERNUNGSWERKZEUG FÜR EINEN FINGERGRIFF-LUER-ADAPTER
RÉCIPIENT DE COLLECTE D'ÉCHANTILLON DE FLUIDE AVEC CAPUCHON ET OUTIL DE RETRAIT POUR ADAPTATEUR LUER DE PRÉHENSION DE DOIGT

(30) Priority: 01.07.2020 US 202063047063 P
(43) Date of publication of application: 10.05.2023
(60) Divisional application: 25184673.9
(73) Proprietor: Becton, Dickinson and Company, Franklin Lakes, NJ 07417 (US)
(72) Inventor: BAKER, Michael, Oradell, New Jersey 07649 (US); FELTYBERGER, Daniel, Sparta, New Jersey 07871 (US); STAYDUHAR, Elizabeth, Charlestown, Massachusetts 02129 (US); WIGHT, David, Colie, West Orange, New Jersey 07052 (US)
(74) Representative: Germain Maureau
(86) International application number: PCT/US2021/039927
(87) International publication number: WO 2022/006294

(56) References cited:
- WO-A1-2011/073729
- US-A- 4 300 404
- US-A1- 2013 133 132
- US-A1- 2013 175 266

## Description

### BACKGROUND

### Field of the Disclosure

The present disclosure relates generally to a container assembly for collecting a fluid specimen. More particularly, the present disclosure relates to a container assembly including a combined finger grip luer adapter (FGLA) cap and removal tool.

### Description of the Related Art

Documents US 2013/133132 A1, US 4 300 404 A, WO 2011/073729 Al and US 2013/175266 Al disclose container assemblies for collecting a fluid specimen. To conduct laboratory testing on biological fluid samples such as urine, it is necessary to provide a container for collecting the fluid sample. These collection containers typically include a cup-shaped container with a removable lid. Once a fluid sample has been collected in the container, the lid is reapplied. The collection container may then be transported to a laboratory or other testing facility where a sample of the collected specimen is extracted for test purposes.

To simplify the sample extraction process, prior collection containers have used lids which not only cover and seal the collection container, but also provide for the use of an extraction device which permits the extraction of a sample of the fluid specimen. Such lids may include a receptable or cavity which supports a tube extending within the cavity to the lower end of the cup-shaped container in fluid communication with the specimen contained within the container. The tube or the lid may include an elongated receptacle housing a cannula and needle so that an air-evacuated collection device (e.g., a specimen collection tube) may be attached thereto to draw a portion of the collected sample thereinto without the need for removal of the lid. In such configurations, the sample can be removed without spilling or contaminating the sample and/or cavity area. Subsequent samples may be drawn from the collection container by using a plurality of collection tubes.

However, in prior collection containers, the cannula is provided within the elongated receptacle in the form of a finger grip luer adapter (FGLA) that is non-removably integrated into the container lid. Thus, when the user has completed all sample collection(s) and wishes to dispose of the container assembly, the entire removable lid must be disposed of in an appropriate sharps container or sharps biohazard container. Due to their large volume, removable lids will quickly fill the sharps container, necessitating frequent service by a qualified disposal firm to safely handle the sharps and/or biohazard waste. As such disposal services are far more expensive than conventional waste disposal services, health care providers generally seek to minimize the accumulated volume of sharps and/or biohazard waste.

Additionally, due to the FGLA being integrated into the lid, the patient and/or healthcare worker may be more at risk of needle-stick injuries during sample collection, transport, and/or disposal.

### SUMMARY

In view of the foregoing, there exists a need for a fluid sample collection container having a reduced volume of sharps waste and which protects users and/or healthcare workers from potential needle-stick injuries.

In accordance with an embodiment of the present invention, a container assembly for collecting a fluid specimen includes a collection container defining a chamber for receiving the fluid specimen, and a container lid couplable to an open end of the collection container to at least partially close the open end thereof. The container assembly also includes an elongate receptacle extending from the container lid into the chamber of the collection container when the container lid is coupled to the open end of the collection container, the elongate receptacle having an open end portion defined within the container lid and configured to receive a specimen collection tube therein. The container assembly also includes a finger grip luer adapter removably coupled to the container lid within the elongate receptacle, with the finger grip luer adapter including a needle extending therefrom. The container assembly further includes an elongate cap removably coupled to the finger grip luer adapter and configured to extend over at least the needle of the finger grip luer adapter, wherein an upper portion of the elongate cap extends above at least a portion of the open end portion of the elongate receptacle when coupled to the finger grip luer adapter.

In certain configurations, the upper portion of the elongate cap include a finger grip portion, and the finger grip portion is configured to extend above an upper surface of the container lid when coupled to the finger grip luer adapter. The finger grip portion may include a ribbed or textured surface so as to improve a user's grip on the finger grip portion of the elongate cap. The elongate cap may include an elongate portion and a cavity, wherein the cavity extends from an open end on a distal end of the elongated portion. The distal end of the elongated portion of the elongate cap may be configured to slip-fittingly or press-fittingly couple to an upper portion of the finger grip luer adapter.

In certain embodiments, the upper portion of the finger grip luer adapter includes a ribbed or textured surface. In other configurations, the lower portion of the finger grip luer adapter is configured to be removably couplable to the elongate receptacle of the container lid. The lower portion of the finger grip luer adapter may include external threads, and the external threads may be configured to mate with a corresponding internal thread portion of the elongate receptacle. The finger grip luer adapter may be threadingly coupled to the container lid.

In other configurations, the finger grip luer adapter is removable from the container lid by way of counterclockwise rotation of the elongate cap when the elongate cap is coupled to the finger grip luer adapter. At least one of the collection container and the container lid are formed of a polymeric resin. Optionally, the elongate cap is formed of a polymeric resin.

In accordance with a further embodiment of the present invention, a container lid assembly for use with a container assembly for collecting a fluid specimen, includes an elongate receptacle having an open end portion defined within the container lid and configured to receive a specimen collection tuber therein. The container lid assembly also includes a finger grip luer adapter removably coupled to the container lid within the elongate receptacle, the finger grip luer adapter comprising a needle extending therefrom, and an elongate cap removably coupled to the finger grip luer adapter. The elongate cap is configured to extend over at least the needle of the finger grip luer adapter, in which an upper portion of the elongate cap extends above at least a portion of the open end portion of the elongate receptacle when coupled to the finger grip luer adapter.

In certain configurations, the distal end of the elongate cap is configured to slip-fittingly or press-fittingly couple to an upper portion of the finger grip luer adapter. The upper portion of the finger grip luer adapter may include a ribbed or textured surface. The lower portion of the finger grip luer adapter may be configured to be removably couplable to the elongate receptacle of the container lid. Optionally, the lower portion of the finger grip luer adapter may include external threads, which are configured to mate with a corresponding internal thread portion of the elongate receptacle. The finger grip luer adapter may be removable from the container lid by way of counterclockwise rotation of the elongate cap when the elongate cap is coupled to the finger grip luer adapter.

In accordance with yet another embodiment of the present invention, a method of forming a container assembly for collecting a fluid specimen includes providing a collection container defining a chamber for receiving the fluid specimen. The method also includes providing a container lid couplable to the collection container, wherein the container lid comprises an elongate receptacle having an open end portion defined within the container lid and configured to receive a specimen collection tube therein. The method also includes coupling a finger grip luer adapter to the container lid within the elongate receptacle, the finger grip luer adapter having a needle extending therefrom. The method further includes coupling an elongate cap to the finger grip luer adapter such that the elongate cap extends over at least the needle of the finger grip luer adapter, wherein an upper portion of the elongate cap extends above at least a portion of the open end portion of the elongate receptacle when coupled to the finger grip luer adapter.

In certain configurations, the method further requires that the finger grip luer adapter is threadingly coupled to the elongate receptacle of the container lid.

Further details and advantages of the present disclosure will be understood from the following detailed description read in conjunction with the accompanying drawings.

### BRIEF DESCRIPTION OF THE DRAWINGS

FIG. 1 is a perspective view of a fluid sample collection container assembly in accordance with an aspect of the present disclosure;
FIG. 2 is a cross-sectional view of the fluid sample collection container assembly having a FGLA cap and removal tool in accordance with an aspect of the present disclosure;
FIG. 3A is a perspective view of the fluid sample collection container assembly and FGLA cap and removal tool in a first use configuration;
FIG. 3B is a perspective view of the fluid sample collection container assembly and FGLA cap and removal tool in a second use configuration;
FIG. 3C is a perspective view of the fluid sample collection container assembly and FGLA cap and removal tool in a third use configuration;
FIG. 3D is a perspective view of the fluid sample collection container assembly and FGLA cap and removal tool in a fourth use configuration; and
FIG. 4 is a cross-sectional perspective view of a container lid assembly for use with a fluid sample collection container assembly, FGLA cap and removal tool in accordance with another aspect of the disclosure.

### DESCRIPTION OF THE INVENTION

The following description is provided to enable those skilled in the art to make and use the described aspects contemplated for carrying out the invention. The scope of the invention is defined by the independent claims. Preferred embodiments are defined in the dependent claims.

For the purposes of the description hereinafter, the terms "upper", "lower", "right", "left", "vertical", "horizontal", "top", "bottom", "lateral", "longitudinal", and derivatives thereof shall relate to the invention as it is oriented in the drawings. However, it is to be understood that the invention may assume various alternative variations, except where expressly specified to the contrary. It is also to be understood that the specific devices illustrated in the attached drawings, and described in the following specification, are simply exemplary aspects of the invention. Hence, specific dimensions and other physical characteristics related to the aspects disclosed herein are not to be considered as limiting.

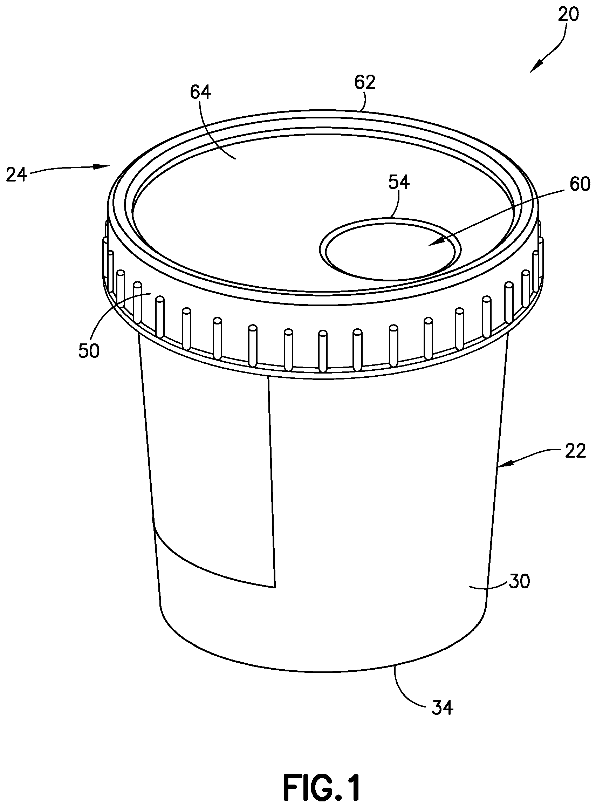

Referring to FIG. 1, a fluid sample collection container assembly **20** in accordance with an aspect of the present disclosure is shown. Fluid sample collection container assembly **20** includes a collection container **22** and a container lid **24.** An exemplary collection container assembly **20** in accordance with the present disclosure may be used to safely collect a fluid specimen (e.g., urine), transport the fluid specimen, and draw a sample of the fluid specimen.

In one embodiment, collection container **22** and container lid **24** may be formed from any conventional material such as, e.g., a polymeric resin. Polymeric resins are well known in the art and include, for example, polyethylene, polycarbonate, polystyrene, and similar polymeric resinous materials. However, it is to be understood that collection container **22** and/or container lid **24** may be formed of other appropriate materials, and may be formed of different materials.

Collection container **22** generally includes a sidewall **30** extending between a first, open end (not shown) and a second, closed end **34.** Sidewall **30** defines an interior chamber for receiving a fluid specimen such as, e.g., urine. In one embodiment, sidewall **30** of collection container **22** comprises a slightly tapering, tubular vessel having continuous, tapered sidewalls **30**. In one embodiment, the collection chamber of container **22** is suitable for holding biologically hazardous materials. In one embodiment, sidewall **30** of collection container **22** may include at least one fill level indicator which identifies a fluid level of a collected fluid specimen.

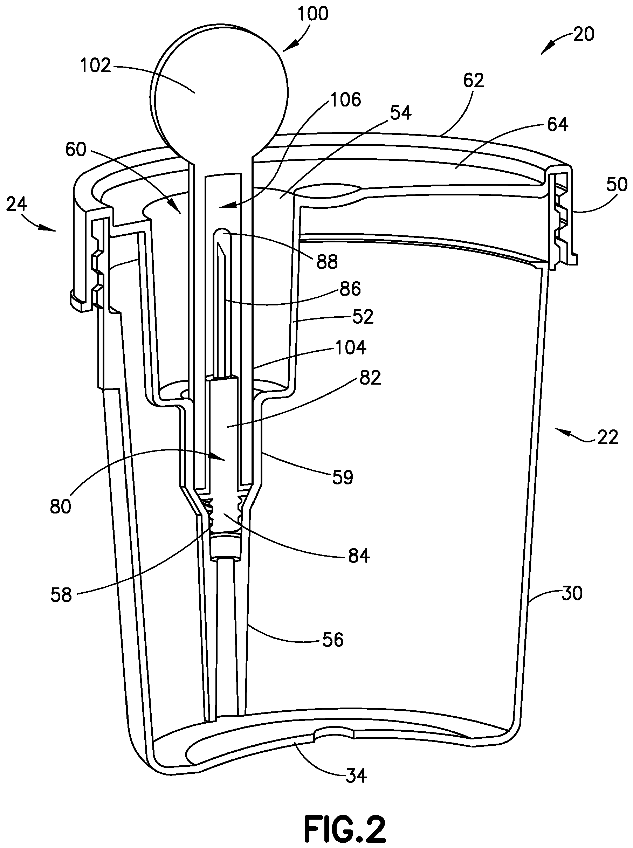

Container lid **24** generally includes a flange **50** extending around its outer rim, with flange **50** being sized to provide a tight fit upon the collection container **22** when container lid **24** is placed over the first, open end of collection container **22.** In one embodiment, container lid **24** includes a generally disc-shaped component having an outer or peripheral zone **62** and an inner or central zone **64.** Flange **50** extends downward from peripheral zone **62** of container lid **24.** As is shown in FIG. 2, flange **50** includes an inner surface which includes a means or mechanism for sealingly engaging container lid **24** with the collection container **22.** In one embodiment, flange **50** includes an interior threaded portion, thereby enabling container lid **24** to be threadingly connectable to a corresponding exterior threaded portion of the collection container **22.** In other embodiments, the sealing portion of container lid **24** may include a snap fit mechanism, a ball detent, an interference fit mechanism, locking tabs, a spring loaded locking mechanism, a latch, or other similar mechanism to sealingly engage container lid **24** to collection container **22,** thereby substantially preventing a fluid specimen contained within collection container **22** and container lid **24** from leaking out, while also preventing contaminants from entering.

Referring to FIG. 2, container lid **24** also includes an elongate receptacle **52** extending from the central zone **64** into collection container **22** and towards the second, closed end **34** of collection container **22.** Receptacle **52** includes an open end portion **54** and an opposing lower end portion **56,** with a receiving cavity **60** defined therein. In one embodiment, receiving cavity **60** is sized and shaped to receive a specimen collection tube such as, e.g., an evacuated tube (not shown). In this way, a fluid specimen within collection container **22** can be transferred to the specimen collection tube without the need to remove container lid **24.**

Referring still to FIG. 2, a finger grip luer adapter (FGLA) **80** is positioned within a central portion **59** of the receptacle **52.** Specifically, FGLA **80** includes an upper portion **82** and a lower portion **84.** Upper portion **82** is configured to extend upward toward receiving cavity **60** and may include ribbed or textured outer sidewalls. On the other hand, in one embodiment, lower portion **84** includes external threads, which are configured to mate with a corresponding internal thread portion **58** of the receptacle **52.** In this way, the FGLA **80** may be removably secured to the receptacle **52** via a threaded interface. Alternatively, in another embodiment, FGLA **80** may be removably secured to receptacle **52** by way of a press-fit or slip-fit connection.

Additionally, FGLA **80** includes a needle **86** extending upward from upper portion **82** and into the receiving cavity **60**. In one embodiment, a retractable sleeve **88** may extend over needle **86.** While not shown, needle **86** is configured to pierce the stopper of a specimen collection tube that is inserted into receptacle **52** by a user. In this way, a fluid specimen may be drawn from the collection container **22** into the specimen collection tube through the lower end portion **56** of receptacle **52** and FGLA **80**, thereby enabling the user to collect the fluid specimen without necessitating removal of the container lid **24** from collection container **22.**

FIG. 2 further shows a combined FGLA cap and removal tool **100** in accordance with an aspect of the present disclosure. As will be described in detail hereinbelow, FGLA cap and removal tool **100** serves the dual purpose of protecting all users from needle-stick injuries and substantially reducing sharps waste when the fluid sample collection container assembly **20** is to be discarded.

In one embodiment, FGLA cap and removal tool **100** includes a finger grip portion **102.** Finger grip portion **102** is configured to extend outside of the receiving cavity **60** and above the central zone **64** of container lid **24** to allow for easy access by the user. Additionally, in one embodiment, the finger grip portion **102** may be ribbed, textured, or otherwise treated so as to improve the user's hold on the finger grip portion **102.** The FGLA cap and removal tool **100** may be formed of any appropriate material or materials such as, e.g., a polymeric resin. Examples of polymeric resins include, e.g., polyethylene, polycarbonate, polystyrene, and similar polymeric resinous materials. However, it is to be understood that FGLA cap and removal tool **100** may be formed of other appropriate materials, and may be formed of two or more different materials.

FGLA cap and removal tool **100** also includes an elongated bottom portion **104.** A distal end of the elongated portion **104** is open, and a cavity **106** is formed within the elongated portion **104.** The cavity **106** extends from the open end of elongated portion **104** toward the finger grip portion **102.** As is shown in FIG. 2, a lower section of cavity **106** of elongated portion **104** is configured to be slip-fit or press-fit over the upper portion **82** of FGLA **80** such that the elongated portion **104** is removably secured to upper portion **82** of FGLA **80**. As disclosed above, the upper portion **82** may include ribbed or textured outer sidewalls, which may aid in providing a secure interface between the FGLA **80** and the FGLA cap and removal tool **100.** Additionally and/or alternatively, the inner sidewall of elongated portion **104** may also be ribbed or textured. Furthermore, in another embodiment, the lower section of cavity **106** of elongated portion **104** may include a threaded portion configured to mate with a complimentary threaded portion on the upper portion **82** of the FGLA **80**.

As is shown in FIG. 2, the cavity **106** is sized so as extend over the needle **86** of FGLA **80**. In this way, when FGLA cap and removal tool **100** is in position on FGLA **80**, the FGLA cap and removal tool **100** acts to protect all users (e.g., patients and healthcare workers) from potential needle stick injuries, as needle **86** is completely enclosed within cavity **106.** As the FGLA cap and removal tool **100** is slip-fit or press-fit onto FGLA **80**, the user can remove the FGLA cap and removal tool **100** by applying an upward pulling force via the finger grip portion **102.** Conversely, the user can replace the FGLA cap and removal tool **100** over the FGLA **80** by applying a downward pushing force via the finger grip portion **102.**

However, while FGLA cap and removal tool **100** may be slid onto (or removed from) FGLA **80** via axial force relative to the FGLA **80**, the interface between lower section of cavity **106** of elongated portion **104** and the upper portion **82** of FGLA **80** is sufficiently constricted so as to transfer rotational force applied by the user via finger grip portion **102** onto the FGLA **80**. That is, if the user rotates the FGLA cap and removal tool **100** in a counterclockwise direction, lower portion **84** of FGLA **80** will unthread from internal thread portion **58** of receptacle **52.** As the user continues to rotate the FGLA cap and removal tool **100,** the FGLA **80** will eventually disconnect entirely from receptacle **52,** leaving the FGLA **80** secured to only the FGLA cap and removal tool **100.** In this way, the FGLA **80** (and the incorporated needle **86**) are separable from the container lid **24,** with the needle **86** being enclosed within the elongated bottom portion **104** of FGLA cap and removal tool **100.**

Once removed, the user may then dispose of both the FGLA cap and removal tool **100** and the attached FGLA **80** in an appropriate sharps container or sharps biohazard container. As discussed above, conventional container lids typically used in specimen extraction via evacuated tubes include a finger grip luer adapter (FGLA) that is non-removably integrated into the container lid. Thus, the entire lid assembly must be disposed of within a sharps container or sharps biohazard container. However, in accordance with embodiments of the present disclosure, only the FGLA cap and removal tool **100** and removable FGLA **80** need be disposed of in a sharps container or sharps biohazard container, thereby greatly reducing the volume (and subsequent cost) of sharps waste, as the container lid **24** and collection container **22** may be disposed of separately as standard medical waste.

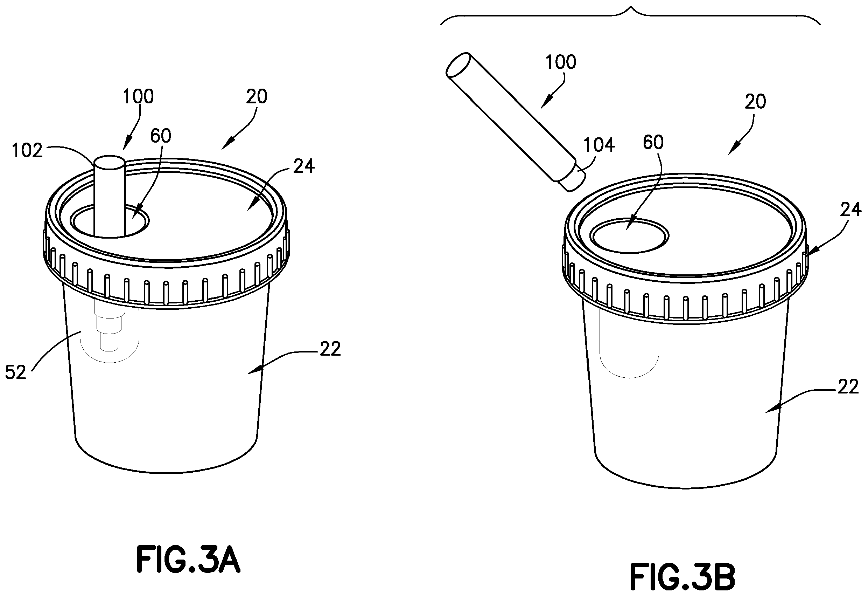

Referring to FIGS. 3A-3D, various in-use views of fluid sample collection container assembly **20** in accordance the present disclosure are shown. FIG. 3A illustrates fluid sample collection container assembly **20** prior to or after fluid sample collection, wherein FGLA cap and removal tool **100** is positioned on the needle and FGLA (not shown). In this way, the FGLA cap and removal tool 100 protects all users from the needle housed within the receiving cavity **60** of container lid **24.**

Referring to FIG. 3B, when the user wishes to the expose the needle and FGLA within receiving cavity **60** in order to collect a fluid sample via, e.g., an evacuated tube, the FGLA cap and removal tool **100** may be pulled upward, thereby releasing the slip-fit or press-fit connection between the FGLA and the elongated portion **104.** After collection of the fluid sample, the FGLA cap and removal tool **100** may be returned to the position shown in FIG. 3A.

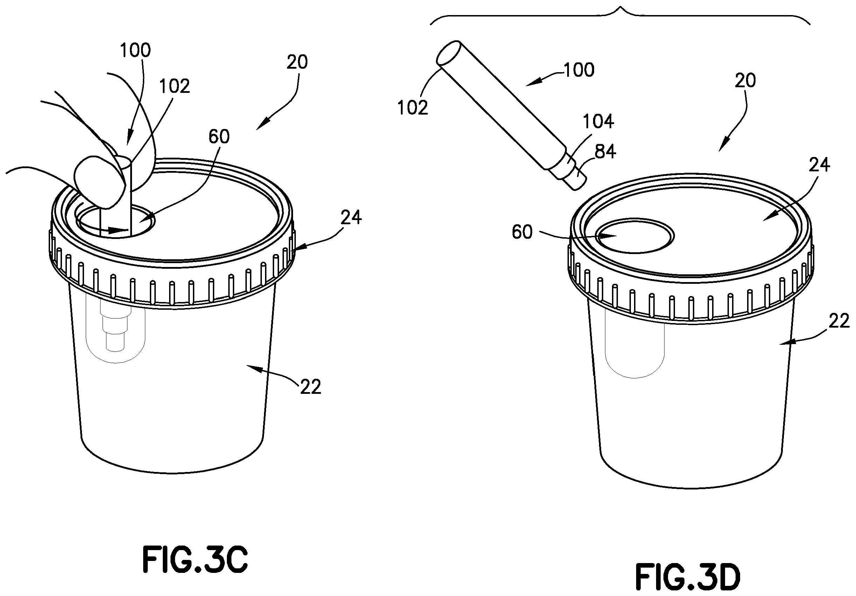

When all necessary fluid sample(s) have been collected from the collection container **22** and the user wishes to discard of the fluid sample collection container assembly **20**, the user may rotate the FGLA cap and removal tool **100** in a counter-clockwise direction, as is shown in FIG. 3C. As described above, such rotation allows for the separation of the FGLA from the container lid **24,** with the needle of the FGLA still being protected within the elongated portion **104** of the FGLA cap and removal tool **100,** as is shown in FIG. 3D. The user may then discard of the combined FGLA cap and removal tool **100** and FGLA into an appropriate sharps container or sharps biohazard container, while the remaining portions of the fluid sample collection container assembly **20** (i.e., the collection container **22** and the container lid **24**) may be disposed of in a standard medical waste container.

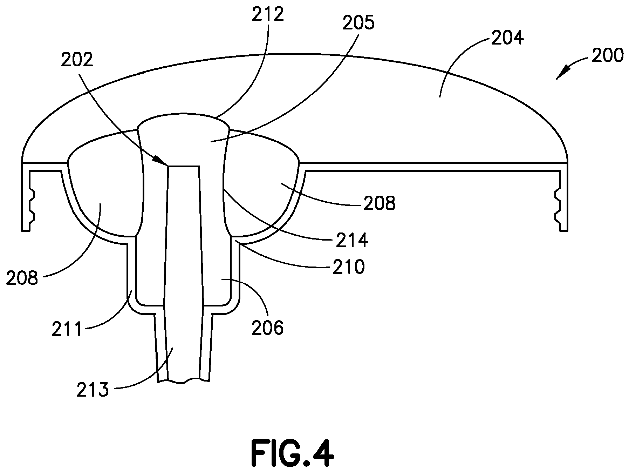

Next, referring to FIG. 4, a container lid assembly **200** for use with a fluid sample collection container assembly in accordance with another aspect of the present disclosure is shown. In the embodiments described above with respect to FIGS. 2-3D, the fluid sample collection container assembly **20** included an FGLA cap and removal tool **100** that at least partially extends above a top surface **64** of the container lid **24,** thereby enabling the user to grip the FGLA cap and removal tool **100** without reaching into the receiving cavity **60**. However, in the embodiment shown in FIG. 4, container lid assembly **200** includes a FGLA cap and removal tool **202**, the uppermost point of which extends below a top surface **204** of the container lid assembly **200**.

Referring still to FIG. 4, the container lid assembly **200** includes an elongate receptacle 211, which is configured to extend into a collection container (not shown). An opening **212** is formed in the top surface **204** of the container lid assembly **200** above the elongate receptacle **211,** while a lower portion 213 extends below the elongate receptacle **211.** While not shown in FIG. 4, is to be understood that the lower portion **213** may be configured to receive a finger grip luer adapter (FGLA), similar to FGLA **80** shown and described with respect to FIG. 2, with the FGLA cap and removal tool **202** configured to extend over portions of the FGLA to protect from needle stick injuries and/or enable removal of the FGLA without needle exposure.

Elongate receptacle **211** includes cut-out portions **208** at an upper end portion thereof, with the cut-out portions **208** defining an open end portion **214** accessible to the fingers of the user. When installed, an upper portion of the FGLA cap and removal tool **202** extends within an upper receiving cavity 205 and above at least a portion of the open end portion **214,** while a lower portion of the FGLA cap and removal tool **202** extends within a lower receiving cavity **206**, which lies below the open end portion **214.** The upper receiving cavity **205** is defined between the opening **212** and a juncture **210** of the elongate receptacle **211,** while the lower receiving cavity **206** is defined between the juncture **210** and the lower surface of the elongate receptacle **211.** When combined, the upper receiving cavity **205** and lower receiving cavity **206** are sized and shaped to receive a specimen collection tube such as, e.g., an evacuated tube (not shown). In this way, a fluid specimen within the collection container can be transferred to the specimen collection tube without the need to remove container lid assembly **200.**

Due to the cut-out portions **208** formed in the container lid assembly **200**, the FGLA cap and removal tool **202** need not include a gripping portion which extends above the top surface **204** in order for a user to access the FGLA cap and removal tool **202**. However, because the needle of the FGLA is housed within the lower receiving cavity **206** of the elongate receptacle 211, the user is substantially protected from needle stick injuries when installing or removing the FGLA cap and removal tool **202**.

While several embodiments of a fluid sample collection container assembly are shown in the accompanying figures and described hereinabove in detail, other embodiments will be apparent to, and readily made by, those skilled in the art providing that they fall within the scope of the appended claims.

## Claims

1. A container assembly (20) for collecting a fluid specimen, comprising:
a collection container (22) defining a chamber for receiving the fluid specimen; and
a container lid (24) couplable to an open end of the collection container (22) to at least partially close the open end thereof,
an elongate receptacle (52) extending from the container lid (24) into the chamber of the collection container (22) when the container lid (24) is coupled to the open end of the collection container (22), the elongate receptacle (52) having an open end portion (54) defined within the container lid (24) and configured to receive a specimen collection tube therein;
a finger grip luer adapter (80) removably coupled to the container lid (24) within the elongate receptacle (52), the finger grip luer adapter (80) comprising a needle (86) extending therefrom; and
an elongate cap (100) removably coupled to the finger grip luer adapter (80) and configured to extend over at least the needle (86) of the finger grip luer adapter (80), wherein an upper portion of the elongate cap (100) extends above at least a portion of the open end portion (54) of the elongate receptacle (52) when coupled to the finger grip luer adapter (80),
**characterized in that**
the finger grip luer adapter (80) is removable from the elongate receptacle (52) through the open end portion (54) of the elongate receptacle (52).

2. The container assembly (20) of claim 1, wherein the upper portion of the elongate cap (100) comprises a finger grip portion (102), and wherein the finger grip portion (102) is configured to extend above an upper surface of the container lid (24) when coupled to the finger grip luer adapter (80).

3. The container assembly (20) of claim 2, wherein the finger grip portion (102) comprises a ribbed or textured surface so as to improve a user's grip on the finger grip portion (102) of the elongate cap (100).

4. The container assembly (20) of claim 1, wherein the elongate cap (100) comprises an elongated portion (104) and a cavity (106), wherein the cavity (106) extends from an open end on a distal end of the elongated portion (104).

5. The container assembly (20) of claim 4, wherein the distal end of the elongated portion (104) of the elongate cap (100) is configured to slip-fittingly or press-fittingly couple to an upper portion (82) of the finger grip luer adapter (80).

6. The container assembly (20) of claim 5, wherein the upper portion (82) of the finger grip luer adapter (80) comprises a ribbed or textured surface.

7. The container assembly (20) of claim 5, wherein a lower portion (84) of finger grip luer adapter (80) is configured to be removably couplable to the elongate receptacle (52) of the container lid (24).

8. The container assembly (20) of claim 7, wherein the lower portion (84) of the finger grip luer adapter (80) comprises external threads, and wherein the external threads are configured to mate with a corresponding internal thread portion of the elongate receptacle (52).

9. The container assembly (20) of claim 1, wherein the finger grip luer adapter (80) is threadingly coupled to the container lid (24).

10. The container assembly (20) of claim 9, wherein the finger grip luer adapter (80) is configured so as to be removable from the container lid (24), with the finger grip luer adapter (80) being removed by way of counterclockwise rotation of the elongate cap (100) when the elongate cap (100) is coupled to the finger grip luer adapter (80).

11. The container assembly (20) of claim 1, wherein at least one of the collection container (22) and the container lid (24) are formed of a polymeric resin.

12. The container assembly (20) of claim 1, wherein the elongate cap (100) is formed of a polymeric resin.

13. A method of forming a container assembly (20) for collecting a fluid specimen, the method comprising:
providing a collection container (22) defining a chamber for receiving the fluid specimen;
providing a container lid (24) couplable to the collection container (22), wherein the container lid (24) comprises an elongate receptacle (52) extending from the container lid (24) into the chamber of the collection container (22) when the container lid (24) is coupled to an open end of the collection container (22), the elongate receptacle (52) having an open end portion (54) defined within the container lid (24) and configured to receive a specimen collection tube therein;
coupling a finger grip luer adapter (80) to the container lid (24) within the elongate receptacle (52), the finger grip luer adapter (80) comprising a needle (86) extending therefrom; and
coupling an elongate cap (100) to the finger grip luer adapter (80) such that the elongate cap (100) extends over at least the needle (86) of the finger grip luer adapter (80), wherein an upper portion of the elongate cap (100) extends above at least a portion of the open end portion (54) of the elongate receptacle (52) when coupled to the finger grip luer adapter (80),
**characterized in that** the finger grip luer adapter (80) is removable from the elongate receptacle (52) through the open end portion (54) of the elongate receptacle (52).

14. The method of claim 13, wherein the finger grip luer adapter (80) is threadingly coupled to the elongate receptacle (52) of the container lid (24).

## Patentansprüche

1. Behälterbaugruppe (20) zum Auffangen einer Flüssigkeitsprobe, umfassend:
einen Auffangbehälter (22), der eine Kammer zum Aufnehmen der Flüssigkeitsprobe definiert; und
einen Behälterdeckel (24), der mit einem offenen Ende des Auffangbehälters (22) gekoppelt werden kann, um dessen offenes Ende mindestens teilweise zu schließen,
eine längliche Aufnahme (52), die sich vom Behälterdeckel (24) in die Kammer des Auffangbehälters (22) erstreckt, wenn der Behälterdeckel (24) mit dem offenen Ende des Auffangbehälters (22) gekoppelt ist, wobei die längliche Aufnahme (52) einen offenen Endabschnitt (54) aufweist, der innerhalb des Behälterdeckels (24) definiert und so eingerichtet ist, dass er ein Probenauffangröhrchen darin aufnimmt;
einen Fingergriff-Luer-Adapter (80), der innerhalb der länglichen Aufnahme (52) abnehmbar mit dem Behälterdeckel (24) gekoppelt ist, wobei der Fingergriff-Luer-Adapter (80) eine sich von dort erstreckende Nadel (86) umfasst; und
eine längliche Kappe (100), die abnehmbar mit dem Fingergriff-Luer-Adapter (80) gekoppelt und so eingerichtet ist, dass sie sich mindestens über die Nadel (86) des Fingergriff-Luer-Adapters (80) erstreckt, wobei sich ein oberer Abschnitt der länglichen Kappe (100) über mindestens einen Abschnitt des offenen Endabschnitts (54) der länglichen Aufnahme (52) erstreckt, wenn sie mit dem Fingergriff-Luer-Adapter (80) gekoppelt ist,
**dadurch gekennzeichnet, dass**
der Fingergriff-Luer-Adapter (80) durch den offenen Endabschnitt (54) der länglichen Aufnahme (52) von der länglichen Aufnahme (52) abnehmbar ist.

2. Behälterbaugruppe (20) nach Anspruch 1, wobei der obere Abschnitt der länglichen Kappe (100) einen Fingergriffabschnitt (102) umfasst, und wobei der Fingergriffabschnitt (102) so eingerichtet ist, dass er sich über eine obere Oberfläche des Behälterdeckels (24) erstreckt, wenn er mit dem Fingergriff-Luer-Adapter (80) gekoppelt ist.

3. Behälterbaugruppe (20) nach Anspruch 2, wobei der Fingergriffabschnitt (102) eine gerippte oder strukturierte Oberfläche umfasst, um den Griff eines Benutzers auf den Fingergriffabschnitt (102) der länglichen Kappe (100) zu verbessern.

4. Behälterbaugruppe (20) nach Anspruch 1, wobei die längliche Kappe (100) einen länglichen Abschnitt (104) und einen Hohlraum (106) umfasst, wobei sich der Hohlraum (106) von einem offenen Ende an einem distalen Ende des länglichen Abschnitts (104) erstreckt.

5. Behälterbaugruppe (20) nach Anspruch 4, wobei das distale Ende des länglichen Abschnitts (104) der länglichen Kappe (100) so eingerichtet ist, dass es rutschfest oder pressfest mit einem oberen Abschnitt (82) des Fingergriff-Luer-Adapters (80) gekoppelt ist.

6. Behälterbaugruppe (20) nach Anspruch 5, wobei der obere Abschnitt (82) des Fingergriff-Luer-Adapters (80) eine geriffelte oder strukturierte Oberfläche umfasst.

7. Behälterbaugruppe (20) nach Anspruch 5, wobei ein unterer Abschnitt (84) des Fingergriff-Luer-Adapters (80) so eingerichtet ist, dass er abnehmbar mit der länglichen Aufnahme (52) des Behälterdeckels (24) gekoppelt werden kann.

8. Behälterbaugruppe (20) nach Anspruch 7, wobei der untere Abschnitt (84) des Fingergriff-Luer-Adapters (80) Außengewinde umfasst, und wobei die Außengewinde so eingerichtet sind, dass sie mit einem entsprechenden Innengewindeabschnitt der länglichen Aufnahme (52) verbunden sind.

9. Behälterbaugruppe (20) nach Anspruch 1, wobei der Fingergriff-Luer-Adapter (80) mit dem Behälterdeckel (24) mittels eines Gewindes gekoppelt ist.

10. Behälterbaugruppe (20) nach Anspruch 9, wobei der Fingergriff-Luer-Adapter (80) so eingerichtet ist, dass er vom Behälterdeckel (24) abnehmbar ist, wobei der Fingergriff-Luer-Adapter (80) durch Drehen der länglichen Kappe (100) gegen den Uhrzeigersinn abgenommen wird, wenn die längliche Kappe (100) mit dem Fingergriff-Luer-Adapter (80) gekoppelt ist.

11. Behälterbaugruppe (20) nach Anspruch 1, wobei mindestens einer des Auffangbehälters (22) und des Behälterdeckelx (24) aus einem Polymerharz ausgebildet sind.

12. Behälterbaugruppe (20) nach Anspruch 1, wobei die längliche Kappe (100) aus einem Polymerharz ausgebildet ist.

13. Verfahren zum Bilden einer Behälterbaugruppe (20) zum Auffangen einer Flüssigkeitsprobe, wobei das Verfahren Folgendes umfasst:
Bereitstellen eines Auffangbehälters (22), der eine Kammer zum Aufnehmen der Flüssigkeitsprobe definiert;
Bereitstellen eines Behälterdeckels (24), der mit dem Auffangbehälter (22) gekoppelt werden kann, wobei der Behälterdeckel (24) eine längliche Aufnahme (52) umfasst, die sich vom Behälterdeckel (24) in die Kammer des Auffangbehälters (22) erstreckt, wenn der Behälterdeckel (24) mit einem offenen Ende des Auffangbehälters (22) gekoppelt ist, wobei die längliche Aufnahme (52) einen offenen Endabschnitt (54) aufweist, der innerhalb des Behälterdeckels (24) definiert und so eingerichtet ist, dass er ein Probenauffangröhrchen darin aufnimmt;
Koppeln eines Fingergriff-Luer-Adapters (80) mit dem Behälterdeckel (24) innerhalb der länglichen Aufnahme (52), wobei der Fingergriff-Luer-Adapter (80) eine sich von dort erstreckende Nadel (86) umfasst; und
Koppeln einer länglichen Kappe (100) mit dem Fingergriff-Luer-Adapter (80), so dass sich die längliche Kappe (100) über mindestens die Nadel (86) des Fingergriff-Luer-Adapters (80) erstreckt, wobei sich ein oberer Abschnitt der länglichen Kappe (100) über mindestens einen Abschnitt des offenen Endabschnitts (54) der länglichen Aufnahme (52) erstreckt, wenn sie mit dem Fingergriff-Luer-Adapter (80) gekoppelt ist,
**dadurch gekennzeichnet, dass** der Fingergriff-Luer-Adapter (80) durch den offenen Endabschnitt (54) der länglichen Aufnahme (52) von der länglichen Aufnahme (52) abnehmbar ist.

14. Verfahren nach Anspruch 13, wobei der Fingergriff-Luer-Adapter (80) mit der länglichen Aufnahme (52) des Behälterdeckels (24) mittels eines Gewindes gekoppelt ist.

## Revendications

1. Ensemble récipient (20) de collecte d'un échantillon de fluide, comprenant :
un récipient de collecte (22) définissant une chambre pour recevoir l'échantillon de fluide ; et
un couvercle de récipient (24) pouvant être couplé à une extrémité ouverte du récipient de collecte (22) pour fermer au moins partiellement son extrémité ouverte,
un réceptacle allongé (52) s'étendant depuis le couvercle de récipient (24) dans la chambre du récipient de collecte (22) lorsque le couvercle de récipient (24) est couplé à l'extrémité ouverte du récipient de collecte (22), le réceptacle allongé (52) ayant une partie d'extrémité ouverte (54) définie dans le couvercle de récipient (24) et configurée pour recevoir un tube de collecte d'échantillon dedans ;
un adaptateur Luer à prise de doigts (80) couplé de manière amovible au couvercle de récipient (24) dans le réceptacle allongé (52), l'adaptateur Luer à prise de doigts (80) comprenant une aiguille (86) s'étendant depuis celui-ci ; et
un capuchon allongé (100) couplé de manière amovible à l'adaptateur Luer à prise de doigts (80) et configuré pour s'étendre sur au moins l'aiguille (86) de l'adaptateur Luer à prise de doigts (80), où une partie supérieure du capuchon allongé (100) s'étend au-dessus d'au moins une partie de la partie d'extrémité ouverte (54) du réceptacle allongé (52) lorsqu'il est couplé à l'adaptateur Luer à prise de doigts (80),
**caractérisé en ce que**
l'adaptateur Luer à prise de doigts (80) peut être retiré du réceptacle allongé (52) à travers la partie d'extrémité ouverte (54) du réceptacle allongé (52).

2. Ensemble récipient (20) selon la revendication 1, dans lequel la partie supérieure du capuchon allongé (100) comprend une partie de prise de doigts (102), et dans lequel la partie de prise de doigts (102) est configurée pour s'étendre au-dessus d'une surface supérieure du couvercle de récipient (24) lorsqu'elle est couplée à l'adaptateur Luer à prise de doigts (80).

3. Ensemble récipient (20) selon la revendication 2, dans lequel la partie de prise de doigts (102) comprend une surface à nervures ou texturée afin d'améliorer la prise de l'utilisateur sur la partie de prise de doigts (102) du capuchon allongé (100).

4. Ensemble récipient (20) selon la revendication 1, dans lequel le capuchon allongé (100) comprend une partie allongée (104) et une cavité (106), où la cavité (106) s'étend depuis une extrémité ouverte sur une extrémité distale de la partie allongée (104).

5. Ensemble récipient (20) selon la revendication 4, dans lequel l'extrémité distale de la partie allongée (104) du capuchon allongé (100) est configurée pour se coupler par ajustement glissant ou ajustement serré à une partie supérieure (82) de l'adaptateur Luer à prise de doigts (80).

6. Ensemble récipient (20) selon la revendication 5, dans lequel la partie supérieure (82) de l'adaptateur Luer à prise de doigts (80) comprend une surface à nervures ou texturée.

7. Ensemble récipient (20) selon la revendication 5, dans lequel une partie inférieure (84) de l'adaptateur Luer à prise de doigts (80) est configurée pour pouvoir être couplée de manière amovible au réceptacle allongé (52) du couvercle de récipient (24).

8. Ensemble récipient (20) selon la revendication 7, dans lequel la partie inférieure (84) de l'adaptateur Luer à prise de doigts (80) comprend des filetages externes, et dans lequel les filetages externes sont configurés pour s'accoupler avec une partie de filetage interne correspondante du réceptacle allongé (52).

9. Ensemble récipient (20) selon la revendication 1, dans lequel l'adaptateur Luer à prise de doigts (80) est couplé par filetage au couvercle de récipient (24).

10. Ensemble récipient (20) selon la revendication 9, dans lequel l'adaptateur Luer à prise de doigts (80) est configuré de manière à pouvoir être retiré du couvercle de récipient (24), l'adaptateur Luer à prise de doigts (80) étant retiré par rotation dans le sens inverse des aiguilles d'une montre du capuchon allongé (100) lorsque le capuchon allongé (100) est couplé à l'adaptateur Luer à prise de doigts (80).

11. Ensemble récipient (20) selon la revendication 1, dans lequel au moins l'un parmi le récipient de collecte (22) et le couvercle de récipient (24) est formé d'une résine polymère.

12. Ensemble récipient (20) selon la revendication 1, dans lequel le capuchon allongé (100) est formé d'une résine polymère.

13. Procédé de formation d'un ensemble récipient (20) de collecte d'un échantillon de fluide, le procédé comprenant :
la fourniture d'un récipient de collecte (22) définissant une chambre pour recevoir l'échantillon de fluide ;
la fourniture d'un couvercle de récipient (24) pouvant être couplé au récipient de collecte (22), où le couvercle de récipient (24) comprend un réceptacle allongé (52) s'étendant depuis le couvercle de récipient (24) dans la chambre du récipient de collecte (22) lorsque le couvercle de récipient (24) est couplé à une extrémité ouverte du récipient de collecte (22), le réceptacle allongé (52) ayant une partie d'extrémité ouverte (54) définie dans le couvercle de récipient (24) et configurée pour recevoir un tube de collecte d'échantillon dedans
le couplage d'un adaptateur Luer à prise de doigts (80) au couvercle de récipient (24) dans le réceptacle allongé (52), l'adaptateur Luer à prise de doigts (80) comprenant une aiguille (86) s'étendant depuis celui-ci ; et
le couplage d'un capuchon allongé (100) à l'adaptateur Luer à prise de doigts (80) de sorte que le capuchon allongé (100) s'étende sur au moins l'aiguille (86) de l'adaptateur Luer à prise de doigts (80), où une partie supérieure du capuchon allongé (100) s'étend au-dessus d'au moins une partie de la partie d'extrémité ouverte (54) du réceptacle allongé (52) lorsqu'il est couplé à l'adaptateur Luer à prise de doigts (80),
**caractérisé en ce que** l'adaptateur Luer à prise de doigts (80) peut être retiré du réceptacle allongé (52) à travers la partie d'extrémité ouverte (54) du réceptacle allongé (52).

14. Procédé selon la revendication 13, dans lequel l'adaptateur Luer à prise de doigts (80) est couplé par filetage au réceptacle allongé (52) du couvercle de récipient (24).
